# EUROPÄISCHE PATENTANMELDUNG

(11) **EP 2 272 833 A1**
(43) Veröffentlichungstag der Anmeldung: **12.01.2011**
(21) Anmeldenummer: 10179278.6
(22) Anmeldetag: 14.08.1998
(51) Int. Cl.: C07D 239/56, A61K 31/505, A61P 25/18, A61P 25/24

(54) **2-(3-(4-(2-t-Butyl-6-trifluormethylpyrimidin-4-yl)piperazin-1-yl)propylmercapto)pyrimidin-4-ol-fumarat**

(30) Priorität: 14.08.1997 DE 19735410
(62) Teilanmeldung aus: 98946343.5
(71) Anmelder: Abbott GmbH & Co. KG, 65205 Wiesbaden (DE)
(72) Erfinder: Blank, Stefan, 67056, Ludwigshafen (DE); Starck, Dorothea, 67059, Ludwigshafen (DE); Treiber, Hans-Jörg, 68782, Brühl (DE); Koser, Stefan, 67061, Ludwigshafen (DE); Schäfer, Bernd, 76889, Dierbach (DE); Thyes, Marco, 67061, Ludwigshafen (DE); Höger, Thomas, 68535, Edingen Neckarhausen (DE)
(74) Vertreter: Reitstötter - Kinzebach

(57) **Zusammenfassung**

Die vorliegende Erfindung betrifft das Fumarsäuresalz von 2-{3-[4-(2-t-Butyl-6-trifluormethylpyrimidin-4-yl)piperazin-1-yl]propylmercapto}pyrimidin-4-ol sowie pharmazeutische Mittel, welche dieses Salz enthalten. Das Salz ist zur Behandlung von Erkrankungen, die auf Dopamin-D₃-Liganden ansprechen, brauchbar. Es besitzt erhöhte Stabilität bei niedrigem pH und ist daher zur oralen Applikation besonders geeignet.

## Beschreibung

Die Erfindung betrifft das Fumarsäuresalz von 2-{3-[4-(2-t-Butyl-6-trifluormethylpyrimidin-4-yl)piperazin-1-yl]propylmercapto}pyrimidin-4-ol und ein pharmazeutisches Mittel, das diese Verbindung enthält. Diese Verbindung besitzt wertvolle therapeutische Eigenschaften und ist insbesondere zur Behandlung von Erkrankungen brauchbar, die auf Dopamin-D₃-Liganden ansprechen.

Die WO 96/02519 beschreibt die erwähnte Verbindung in Form der freien Base der Formel 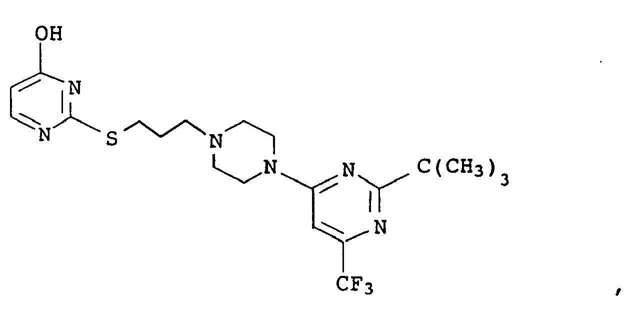 die ebenfalls zur Behandlung von Erkrankungen brauchbar ist, welche auf Dopamin-D₃-Liganden ansprechen. Ein Salz dieser Verbindung ist jedoch nicht offenbart.

Überraschenderweise wurde nun gefunden, daß das Säureadditionssalz dieser Verbindung mit Fumarsäure besondere Vorteile aufweist.

Gegenstand der vorliegenden Erfindung ist daher das Fumarsäuresalz von 2-{3-[4-(2-t-Butyl-6-trifluormethylpyrimidin-4-yl)piperazin-1-yl]propylmercaptolpyrimidin-4-ol sowie ein pharmazeutisches Mittel, welches diese Verbindung enthält.

Gegenstand der Erfindung sind auch die tautomeren Formen (Pyridonstruktur) sowie die Solvate und Hydrate des Fumarsäuresalzes.

Das Fumarsäuresalz besitzt sehr gute Affinität und hohe Selektivität gegenüber dem D₃-Rezeptor, d.h. es handelt sich um einen selektiven Dopamin-D₃-Rezeptor-Liganden, der regioselektiv im limbischen System angreift. Es besitzt eine Selektivität Ki D2/Ki D₃ von 120 (vgl. WO 96/02519). Die Verbindung ist daher zur Behandlung von Erkrankungen brauchbar, die auf Dopamin-D₃-Liganden ansprechen, z.B. zur Behandlung von Erkrankungen des zentralen Nervensystems, insbesondere Schizophrenie, Depressionen, Neurosen und Psychosen. Außerdem ist sie zur Behandlung von Schlafstörungen, Übelkeit und als Antihistaminikum brauchbar. Untersuchungen zur Löslichkeit der Substanzen ergaben eine wesentlich höhere Löslichkeit des Salzes in Wasser im Vergleich zur freien Base. Dies ist für die Resorption bei oraler Anwendung und insbesondere bei parenteraler Anwendung von entscheidendem Vorteil.

Darüber hinaus ist das Fumarat in polaren Lösungsmitteln, wie C₁-C₆-Alkanolen, besser löslich als die freie Base. Aufgrund des veränderten Löslichkeitsverhaltens läßt es sich auch einfacher unter Vermeidung physiologisch nicht akzeptabler Lösungsmittel reinigen.

Die freie Base kann nach den in der WO 96/02519 beschriebenen allgemeinen Verfahren, vorzugsweise nach dem Verfahren (ii) hergestellt werden. Das Fumarat ist erhältlich durch Umsetzung der freien Base mit Fumarsäure in einem geeigneten Lösungsmittel, wie C₁-C₆-Alkanole, insbesondere Methanol, Ethanol, n-Propanol, Isopropanol und n-Butanol, einem Gemisch aus Wasser und einem der erwähnten Alkohole oder einem Ester, wie Essigsäureethylester. Im allgemeinen wird man bei erhöhter Temperatur arbeiten, damit das gewünschte Fumarat beim Abkühlen auskristalliert und auf einfache Weise isoliert werden kann. Die Fumarsäure wird im allgemeinen in äquimolaren Mengen oder mit einem geringen Überschuß von bis zu ca. 10 % zugegeben. Das Fumarat fällt auf diese Weise bereits in hoher Reinheit an. Es kann noch zusätzlich durch Ausrühren oder Umkristallisieren aus einem geeigneten Lösungsmittel, z. B. Wasser, einem der oben genannten Alkanole, Ester oder Gemischen davon, gereinigt werden.

Zur Behandlung der oben erwähnten Erkrankungen wird die erfindungsgemäße Verbindung in üblicher Weise oral oder parenteral (subkutan, intravenös, intramuskulär, intraperitoneal) verabreicht. Orale Verabreichung ist bevorzugt.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, welche die erfindungsgemäße Verbindung enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung ohne sie zu begrenzen.

### Beispiel 1

### Herstellung des Fumarsäuresalzes von 2-{3-[4-[2-t-Butyl-6-trifluormethylpyrimidin-4-yl)-piperazin-1-yl]-propylmercapto]-pyrimidin-4-ol

### 2-t-Butyl-4-hydroxy-6-trifluormethyl-pyrimidin(1)

Die Ausgangsmaterialien sind literaturbekannt.

50 g (0,37 mol) 2,2-Dimethylpropanimidamid-hydrochlorid, gelöst in 200 ml Ethanol, wurden bei Raumtemperatur mit 66,6 g (0,37 mol) Natriummethylat (30%ig in Methanol) und nach weiteren 30 Minuten mit 52 g (0,28 mol) Trifluoracetessigsäureethylester versetzt. Nach 17 Stunden Rühren unter Rückfluß entfernte man das Lösungsmittel im Vakuum, versetzte den Rückstand mit 200 ml Wasser, acidifizierte bis pH 4 und isolierte den auskristallisierten Feststoff mittels Filtration.

| | |
|---|---|
| Ausbeute: 62,2 g | (98 % d.Th.) |
| C₉H₁₁F₃N₂O (MW 220) | Fp. 187-188°C |

### 2-t-Butyl-4-chlor-6-trifluormethylpyrimidin (2)

Zu einer Lösung von 60 g (0,27 mol) 2-t-Butyl-4-hydroxy-6-trifluormethylpyrimidin in 800 ml Dichlormethan wurden zunächst 86,5 ml Thionylchlorid, dann 8 ml DMF getropft und anschließend wurde zum Sieden unter Rückfluß erhitzt. Man entfernte die flüchtigen Bestandteile im Vakuum, nahm den Rückstand in 100 ml Dichlormethan auf, stellte mit gesättigter NaHCO₃-Lösung auf pH 7 ein und er- - hielt nach extraktiver Aufarbeitung 67 g (96 %) eines klaren Öles.

| | |
|---|---|
| Ausbeute 67 g | (96 % d. Th.) |
| C₉H₁₀ClF₃N₂ (MW 239) | |

### 2-t-Butyl-4-[piperazin-1-yl]-6-trifluormethyl-pyrimidin (3)

Zu einer Lösung von 129 g (1,5 mol) Piperazin in 500 ml Ethanol wurde eine Lösung von 60 g (0,25 mol) des oben beschriebenen Chlorpyrimidins, in 200 ml Ethanol bei Siedetemperatur innerhalb 2 h zugetropft und anschließend wurde weitere 6 h bei Siedetemperatur gerührt. Nach beendeter Reaktion wurde das Lösungsmittel im Vakuum entfernt und der Rückstand mit 2 1 Wasser versetzt. Das Produkt kristallisierte beim Abkühlen aus und wurde anschließend abgesaugt.

| | |
|---|---|
| Ausbeute: 56 g | (77 % d.Th.) |
| C₁₃H₁₉ClF₃N₄ (MW 288) | Fp. 78-80°C |

1H-NMR (250 MHz, CDCl₃): δ = 1,3 (s, 9H); 1,8 (s, 1H); 3,0 (m, 4H); 4,7 (m, 4H); 6,6 (s, 1H) ppm.

### 2-t-Butyl-4-[4-(3-chlorpropyl)-piperazin-1-yl]-6-trifluormethylpyrimidin (4)

Zu einer Mischung von 35,1 g (0,22 mol) 1-Brom-3-chlor-propan und 16,0 g (0,16 mol) Triethylamin in 90 ml THF wurden in der Siedehitze 43,2 g (0,15 mol) des vorstehend beschriebenen Piperazins, gelöst in 130 ml THF, getropft und 8 Stunden bei dieser Temperatur gerührt. Nach Abkühlen auf 4°C wurde von den anorganischen Salzen abfiltriert, die THF-Phase im Vakuum eingedampft und der Rückstand aus Isopropanol umkristallisiert.

| | |
|---|---|
| Ausbeute: 32,1 g | (61 % d.Th.) |
| C₁₆H₂₄ClF₃N₄ (MW 365) | Fp. 83-84°C |

1H-NMR (250 MHz, CDCl₃): δ = 1,3 (s, 9H); 1,9 (q, 2H); 2,5 (m, 6H); 3,7 (t, 2H); 3,8 (m, 4H); 6,6 (s, 1H) ppm.

### 2-{3-[4-[2-t-Butyl-6-trifluormethylpyrimidin-4-yl)-piperazin-1-yl]-propylmercapto}-pyrimidin-4-ol (5)

8,4 g (0,066 mol) Thiouracil, 1,6 g (0,066 mol) Lithiumhydroxid und 1,0 g (0,066 mol) Natriumjodid wurden in 200 ml DMF gelöst und auf 100°C erwärmt. Bei dieser Temperatur wurden 20,1 g (0,055 mol) der vorstehend beschriebenen Chlorbase, gelöst in 50 ml DMF, zugegeben und 30 min bei 100°C nachgerührt. Anschließend wurde mit 300 ml Natriumchloridlösung versetzt und 2 mal mit 200 ml Essigsäureethylester extrahiert. Die organische Phase wurde mit Natriumsulfat getrocknet und nach Filtration unter Vakuum eingedampft.

Der Rückstand wurde säulenchromatographisch (Kieselgel, Dichlormethan mit 1 - 4 % Methanol) gereinigt.

| | |
|---|---|
| Ausbeute: 18 g | (72 % d. Th). |
| C₂₀H₂₇F₃N₆OS(MW 457) | Fp. 138-140°C |

1H-NMR (270 MHz, DMSO-d₆):δ=1,3 (s, 9H); 1,8 (q, 2H); 2,4 (m, 6H); 3,3 (t, 2H); 3,75 (m, 4 H); 6,1 (d, 2H); 7,1 (s, 1H); 7,9 (2, 2H) ppm.

### Herstellung des Fumarates (6):

4,56 g (0,01 mol) der vorstehend beschriebenen Base wurden in 25 ml Isopropanol heiß gelöst und eine heiße Lösung von 1,16 g (0,01 mol) Fumarsäure in 15 ml Isopropanol wurde zugegeben. Die beim Abkühlen auskristallisierte Substanz wurde abfiltriert, wobei 4,4 g der Titelverbindung als farblose Kristalle erhalten wurden.

| | |
|---|---|
| Ausbeute: 4,4 g | (76 % d. Th.) |
| C₂₀H₂₇F₃N₆OS x C₄H₄O₄ | (MW 573) Fp. 200 - 202°C |

1H-NMR (250 MHz, DMSO-d₆): δ = 1,3 (s, 9H); 1,9 (q, 2H); 2,5 (m, 6H); 3,2 (t, 2H); 3,8 (mbr, 6H); 6,2 (d, 2H); 6,7 (s, 2H); 7,1 (s, 1H); 7,9 (d, 2H).

## Patentansprüche

1. Hydrate und Solvate des Fumarsäuresalzes von 2-{3-[4-(2-t-Butyl-6-trifluormethylpyrimidin-4-yl)piperazin-1-yl]propylmercapto}pyrimidin-4-ol der Formel

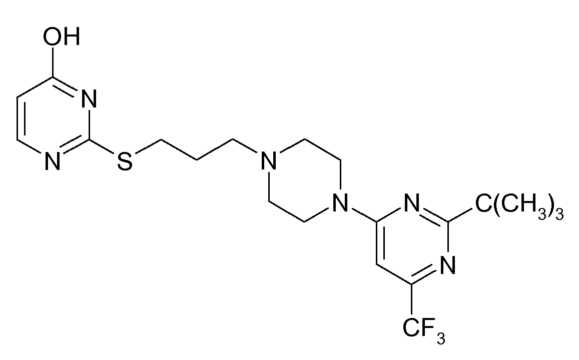

2. Pharmazeutisches Mittel, enthaltend ein Hydrat oder Solvat des Fumarsäuresalzes nach Anspruch 1, gegebenenfalls zusammen mit physiologisch akzeptablen Trägern und/oder Hilfsstoffen.

3. Verwendung von Hydraten oder Solvaten des Fumarsäuresalzes nach Anspruch 1 zur Herstellung eines pharmazeutischen Mittels zur Behandlung von Erkrankungen, die ausgewählt sind unter Schizophrenie, Depressionen, Neurosen, Psychosen, Schlafstörungen und Übelkeit, oder als Antihistaminikum.
